# EUROPÄISCHE PATENTSCHRIFT

(11) **EP 1 996 242 B1**
(45) Veröffentlichungstag und Bekanntmachung des Hinweises auf die Patenterteilung: **13.04.2011**
(21) Anmeldenummer: 07712357.8
(22) Anmeldetag: 28.02.2007
(51) Int. Cl.: A61L 15/46

(54) **SUPERABSORBER MIT VERBESSERTER GERUCHSINHIBIERUNG**
SUPER-ABSORBER HAVING IMPROVED SMELL-INHIBITION
SUPERABSORBANT PRÉSENTANT UNE CAPACITÉ ACCRUE D'INHIBITION DES ODEURS

(30) Priorität: 10.03.2006 EP 06110962
(43) Veröffentlichungstag der Anmeldung: 03.12.2008
(73) Patentinhaber: BASF SE, 67056 Ludwigshafen (DE)
(72) Erfinder: BRAIG, Volker, 69469 Weinheim-Lützelsachsen (DE); SERVAY, Thomas, 67550 Worms (DE)
(86) Internationale Anmeldenummer: PCT/EP2007/051868
(87) Internationale Veröffentlichungsnummer: WO 2007/104641

(56) Entgegenhaltungen:
- EP-A- 1 358 894
- WO-A-03/051413
- DE-A1-102004 033 206
- JP-A- 63 315 200
- JP-A- 2005 168 525

## Beschreibung

Die vorliegende Erfindung betrifft Superabsorber mit verbesserter Geruchsinhibierung, Verfahren zu ihrer Herstellung und ihre Verwendung.

Superabsorber sind bekannt. Für derartige Materialien sind auch Bezeichnungen wie "hochquellfähiges Polymer" "Hydrogel" (oft auch für die trockene Form verwendet), "Hydrogel bildendes Polymer", "Wasser absorbierendes Polymer", "absorbierendes gelbildendes Material", "quellfähiges Harz", "wasserabsorbierendes Harz" oder ähnliche gebräuchlich. Es handelt sich dabei um vernetzte hydrophile Polymere, insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether, vernetzte Carboxymethylcellulose, teilweise vernetztes Polyalkylenoxid oder in wässrigen Flüssigkeiten quellbare Naturprodukte, wie beispielsweise Guarderivate, wobei wasserabsorbierende Polymere auf Basis teilneutralisierter Acrylsäure am weitesten verbreitet sind. Die wesentlichen Eigenschaften von Superabsorbern sind ihre Fähigkeiten, ein Vielfaches ihres Eigengewichts an wässrigen Flüssigkeiten zu absorbieren und die Flüssigkeit auch unter gewissem Druck nicht wieder abzugeben. Der Superabsorber, der in Form eines trockenen Pulvers eingesetzt wird, wandelt sich bei Flüssigkeitsaufnahme in ein Gel, bei der üblichen Wasseraufnahme entsprechend in ein Hydrogel um. Das mit weitem Abstand wichtigste Einsatzgebiet von Superabsorbern ist das Absorbieren von Körperflüssigkeiten. Superabsorber werden beispielsweise in Windeln für Kleinkinder, Inkontinenzprodukten für Erwachsene oder Damenhygieneprodukten verwendet. Andere Anwendungsgebiete sind beispielsweise die als wasserzurückhaltende Mittel im landwirtschaftlichen Gartenbau, als Wasserspeicher zum Schutz vor Feuer, zur Flüssigkeitsabsorption in Lebensmittelverpackungen oder ganz allgemein zur Absorption von Feuchtigkeit.

Finden Superabsorber im Hygienebereich Anwendung, so werden sie Körperflüssigkeiten wie z. B. Urin oder Menstruationsblut ausgesetzt. Derartige Körperflüssigkeiten enthalten stets unangenehm riechende Komponenten wie Amine, Fettsäuren und sonstige organische Komponenten, die für unangenehme Körpergerüche verantwortlich sind. Ein weiteres Problem bei derartigen Hygieneprodukten ist, dass die Körperflüssigkeiten bis zur Entsorgung des Hygieneprodukts über ein gewissen Zeitraum darin verbleiben und durch bakteriellen Abbau von in den zu absorbierten Körperflüssigkeiten enthaltenen stickstoffhaltigen Verbindungen, beispielsweise Harnstoff in Urin, Ammoniak oder auch andere Amine entstehen, die ebenso zu einer merklichen Geruchsbelästigung führen. Nachdem entsprechend häufiges Wechseln des Hygieneprodukts zu erheblichem Aufwand, auch Kostenaufwand, für den Benutzer oder dessen Pflegepersonen führt, sind Hygieneprodukte, bei denen diese Geruchsbelästigung vermieden wird, von Vorteil.

Es sind verschiedene Maßnahmen zur Vermeidung der Geruchsbelästigung bekannt. Gerüche können durch Parfümierung maskiert werden, entstehender Ammoniak oder Amine können durch Absorption oder Reaktion entfernt werden, und der mikrobielle Abbau kann beispielsweise durch Biozide oder Ureaseinhbitoren inhibiert werden. Diese Maßnahmen können einerseits auf den Superabsorber und andererseits auf den Hygieneartikel angewendet werden.

So lehrt etwa EP 1 358 894 A1 Hygieneartikel, die eine Reihe von geruchsverhindernden Zusätzen enthalten können, insbesondere Anhydridgruppen, Säuregruppen, Cyclodextrine, Biozide, Tenside mit einem HLB-Wert kleiner 11, Absorptionsmittel wie Zeolithe, Ton, Aktivkohle, Siliziumdioxid oder aktiviertes Aluminiumoxid, Mikroorganismen, die als Antagonisten zu unerwünschten geruchsbildenden Mikroorganismen wirken, pH-Puffer oder Chelatisierungsmittel. In WO 03/076 514 A2 findet sich eine umfassende Übersicht über die bekannten Maßnahmen zur Vermeidung unangenehmer Gerüche. Unter anderem wird die Verwendung von Bioziden wie Bronopol oder Glyoxylsäure offenbart. Zudem lehrt diese Schrift einen Superabsorber, der Anhydridgruppen enthält, die mit Ammoniak oder Aminen reagieren und diese dadurch in nichtflüchtiger Form binden können.

Ammoniak und Amine werden bei niedrigen pH-Werten als geruchsneutrale Ammoniumsalze gebunden, zudem hemmt ein niedriger pH-Wert das Wachstum von Ammoniak bildenden Bakterien. EP 202 127 A2 lehrt dem gemäß Hygieneartikel mit einem pH-Puffer wie etwa einer organischen Säure oder auch säuremodifizierter Cellulose, der den pH-Wert der Haut im Bereich von 3,0 bis 5,5 hält. EP 316 518 A2 betrifft Superabsorber aus teilneutralisierter polymerer organischer Säure, die wahlweise zusätzlich mit teilneutralisierter Zitronensäure versetzt sind, und die im Kontakt mit Flüssigkeit einen pH-Wert zwischen 5,0 und 6,0 aufweisen. WO 03/002 623 A1 lehrt Superabsorber mit einem pH-Wert unterhalb von 5,7. GB 2 296 013 A beschreibt Hygieneartikel, die eine Polylactidschicht enthalten, so dass bei Flüssigkeitskontakt Milchsäure entsteht, wodurch der pH-Wert abgesenkt wird. WO 00/35 502 A1 lehrt Hygieneartikel mit einer Kombination aus einem Puffer mit einem pH-Wert im Bereich von 3,5 - 5,5 und Milchsäurebakterien. Nach WO 00/59 556 A2 wird ein Superabsorber verwendet, der funktionelle Gruppen enthält, die mit Ammoniak oder Aminen reagieren können, insbesondere zyklische Anhydride, Lactide oder Lactone von Hydroxisäuren. WO 01/32 226 A1 offenbart einen mit organischen Säuren versetzten Superabsorber.

EP 894 502 A1 lehrt die Verwendung von Cyclodextrinen als Absorptionsmittel für Ammoniak in Hygieneartikeln. EP 1 034 800 A1 offenbart Hygieneartikel, die neben einem Absorptionsmittel für Ammoniak, insbesondere Aktivkohle, hochoberflächigem Siliziumdioxid, Tonen, Zeolithen, Kieselgur, Chitin, pH-Puffern, Stärke, Cyclodextrin, oder Ionentauschern auch Oxidationsmittel wie Persäuren oder Diacylperoxiden enthalten. WO 91/11 977 A1 betrifft Zeolithe mit einem SiO2/Al2O3-Verhältnissunter 10 als Absorptionsmittel für Gerüche. Gemäß WO 95/26 207 A1 werden dazu Zeolithe mit einer mittleren Partikelgröße von mindestens 200 Mikrometer verwendet.

In EP 1 149 597 A1 wird Chitosan als geruchsinhibierende Komponente von Hygieneartikeln beschrieben. EP 1 149 593 A1 lehrt kationische Polysaccharide, insbesondere Chitinderivate oder Chitosan in Verbindung mit einem pH-Puffer, der den pH-Wert im Bereich von 3,5 bis 6 einstellt.

EP 739 635 A1 lehrt Natriummeta- und -tetraborat als Ureaseinhibitoren in Superabsorbern. Nach WO 94/25 077 A1 wird eine Mischung aus Alkali- oder Erdalkalitetraborat und Borsäure, Zitronensäure, Weinsäure oder Ascorbinsäure als Puffer im pH-Bereich von 7 bis 10 verwendet. WO 03/053486 A1 offenbart Windeln, die Extrakt aus Yuccapalmen als Ureaseinhibitor enthalten. EP 1 214 878 A1 offenbart die Verwendung von Chelatkomplexen zweiwertiger Metallionen wie etwa des Kupferkomplexes der einfach protonierten Ethylendiamintetraacetats als Ureaseinhibitor. WO 95/24173 A2 lehrt mit bakteriziden Schwermetallen wie Silber, Zink oder Kupfer imprägnierte Zeolithe zur Geruchskontrolle.

EP 311 344 A23 betrifft Hygieneartikel, die neben einem pH-Puffer ein Biozid wie Alkylammoniumhalogenide oder Bisguanidine enthalten. EP 389 023 A2 offenbart Hygieneartikel mit einem Zusatz zur Geruchskontrolle, der aus Bioziden oder Absorptionsmitteln, insbesondere Molsieben, gewählt ist. WO 98/26 808 A2 beschreibt Superabsorber, die Cyclodextrine, Zeolithe, Aktivkohle, Kieselgur oder saure salzbildende Substanzen als Absorptionsmittel für Gerüche sowie Biozide, Ureaseinhibitoren und pH-Regulatoren zur Inhibierung der Geruchsbildung enthalten.

Nach WO 98/20 916 A1 wird in Hygieneartikeln ein Superabsorber verwendet, der mit einem Biozid wie Benzalkoniumchlorid oder Chlorhexidin beschichtet ist. Die ältere, vorläufige US-Patentanmeldung vom 27.07.2005, Seriennummer des US Patent and Trademark Office 60/702931, betrifft lagerstabile Superabsorber mit substituierten Thiophosphorsäuretriamiden als Geruchsinhibitor.

Das Vermischen geruchsabsorbierender hochoberflächige Stoffe in ausreichender Menge mit Superabsorbern bei der Herstellung von Hygieneartikeln erniedrigt die Flüssigkeitsabsorptionskapazität der Mischung. Superabsorber, die selbst geruchsinhibierende Eigenschaften aufweisen, sind solche mit niedrigem pH-Wert. Diese sind jedoch erheblich schwieriger herzustellen als Superabsorber mit höheren pH-Werten. Weniger stark neutralisierte Acrylsäure polymerisiert langsamer, und das saure Polymerisat neigt im Gegensatz zu neutralem oder basischerem zum Verkleben, was die notwendige Nachbearbeitung (Zerteilen des Gels, Trocknen, Mahlen, Klassieren) massiv beeinträchtigt. Zudem weisen saure Superabsorber typischerweise ein schlechteres Flüssigkeitsretentionsvermögen unter Druck auf. Die Verwendung von Bioziden oder Antibiotika in Hygieneartikeln, etwa als Zusatz zum Superabsorber, ist nachteilig, da diese Stoffe durch Diffusion in Kontakt mit der Haut des Benutzers treten und dabei ihre Wirkungen nicht nur gegen geruchsbildende Bakterien, sondern auch in unerwünschter Weise entfalten. Zudem führt ihre Verwendung bei Entsorgung der benutzen Hygieneartikel auf üblichem Weg zu einem erheblichen Eintrag von Bioziden oder Antibiotika in die Umwelt, der nicht nur die Funktion von Kläranlagen beeinträchtigt, sondern auch zur Bildung gegen Antibiotika resistenter Bakterienstämme beiträgt. Ähnlich unerwünschte Effekte sind auch mit der Verwendung von bakteriziden Schwermetallen wie Zink, Silber oder Kupfer verbunden.

Es besteht daher weiterhin die Aufgabe, neue, verbesserte oder andere Superabsorber oder Superabsorber enthaltende Zusammensetzungen mit geruchsinhibierenden Eigenschaften oder mit verbesserter Geruchsinhibierung zu finden. Diese sollen zudem lagerstabil sein, sich insbesondere auch bei längerer Lagerung weder verfärben noch ihre geruchsinhibierenden Eigenschaften verlieren. Unerwünschte Nebeneffekte bei Hautkontakt oder Eintrag von Bestandteilen in die Umwelt sollten nicht auftreten. Zudem sollen die Superabsorber oder Zusammensetzungen gute Flüssigkeitsabsorptions- und Retentionseigenschaften aufweisen, insbesondere wünschenswert sind schnelles Anquellvermögen, gute Flüssigkeitstransporteigenschaften im Gelbett bei gleichzeitig hohem Absorptionsvermögen, hohe Gelfestigkeit und gute Elektrolyttoleranz.

Dem gemäß wurden Zusammensetzungen, die Superabsorber und 2-Keto-L-Gulonsäure und/oder 2-Keto-Glutarsäure enthalten, gefunden.

Die erfindungsgemäße Zusammensetzung führt bei Verwendung in einem Hygieneartikel zur Vermeidung oder zumindest Verringerung unangenehmer Gerüche nach Kontakt mit Körperflüssigkeiten. Es wird vermutet, dass die enthaltene Ketosäure, nämlich 2-Keto-L-Gulonsäure und/oder 2-Keto-Glutarsäure Ammoniak durch chemische Reaktion unter Bildung von Imid und/oder Ammoniumsalzen entfernt, was zum beobachteten geruchsverringernden oder -vermeidenden Effekt führt. Zudem senkt die Ketosäure den pH-Wert im Superabsorberbett im Hygieneartikel ab, so dass das Bakterienwachstum gehemmt wird. Das Absorptions- und Retentionsverhalten des Superabsorbers wird durch die Ketosäure nicht signifikant beeinträchtigt. Es ist nicht nötig, aber möglich, saure Superabsorber zu verwenden. Die Ketosäure beeinträchtigt die Lagerstabilität nicht, unerwünschte Effekte bei Hautkontakt oder Eintrag in die Umwelt wurden weder beobachtet noch sind sie zu erwarten.
Die Ketosäure wirkt nicht bakterizid.

Ketosäuren (dies ist eine übliche Kurzform der systematisch korrekteren Bezeichnung "Ketocarbonsäuren") sind eine Untergruppe der Oxocarbonsäuren, nämlich die Carbonsäuren, die außer einer Carboxigruppe eine Ketongruppe, d.h. eine Gruppe der Formel RR'C=O enthalten, wobei mindestens einer der Reste R und R' eine Carboxifunktion trägt, jedoch keiner dieser Reste R oder R' ein Wasserstoffatom ist. Neben den Ketocarbonsäuren sind die Aldehydcarbonsäuren die weitere Untergruppe der Oxocarbonsäuren. Bei Aldehydcarbonsäuren ist einer der beiden Reste R oder R' in der Formel RR'C=O Wasserstoff. Der einfachste Vertreter der Aldehydcarbonsäuren ist die Glyoxylsäure HCO-COOH, der einfachste Vertreter der Ketocarbonsäuren die Brenztraubensäure H₃C-CO-COOH.

Die erfindungsgemäß verwendete Ketosäure ist 2-Keto-L-Gulonsäure, (das L-Enantiomer der 2-Oxo-3,4,5,6-tetrahydroxihexansäure) und/oder 2-Keto-Glutarsäure (2-Oxo-pentan-1,5-disäure).

Die Herstellung von Ketosäuren ist bekannt. Ein gängiger Weg zur Herstellung von alpha-Ketosäuren ist die oxidative Desaminierung von alpha-Aminosäuren. Viele diese Säuren sind gängige Handelsprodukte und werden großtechnisch, auch auf anderen Wegen als der oxidativen Desaminierung hergestellt. Beispielsweise wird 2-Keto-L-Gulonsäure als direkte Vorstufe von Vitamin C (der 2,3-Enol-Form des 2-Keto-L-gulonsäure-1,4-lactons) in sehr großer Menge hergestellt, meist fermentativ aus Sorbit. Es wurde gefunden, dass Ketosäuren, insbesondere alpha-Ketosäuren, zur Verringerung oder Vermeidung unangenehmer Gerüche verwendet werden können, insbesondere durch Ammoniak hervorgerufener unangenehmer Gerüche.

Die Menge der Ketosäure in der erfindungsgemäßen Zusammensetzung, bezogen auf die Superabsorbermenge in der Zusammensetzung, ist im Allgemeinen mindestens 0,005 Gew.-%, vorzugsweise mindestens 0,01 Gew.-%, in besonders bevorzugter Form mindestens 0,1 Gew.-% und in ganz besonders bevorzugter Form mindestens 0,5 Gew.-% sowie im Allgemeinen höchstens 15 Gew.-%, vorzugsweise höchstens 12 Gew.-% und in besonders bevorzugter Form höchstens 10 Gew.-%. Beispielsweise beträgt diese Menge 1 Gew.-%, 2 Gew.-%, 3 Gew.-%, 4 Gew.-%, 5 Gew.-%, 6 Gew.-%, 7 Gew.-%, 8 Gew.-% oder 9 Gew.-%, jeweils bezogen auf die Menge an Superabsorber in der Zusammensetzung. Die Zusammensetzung kann außer Ketosäure und Superabsorber weitere Bestandteile, Zusätze, Hilfsmittel oder sonstige Komponenten enthalten. Vorzugsweise besteht sie im Wesentlichen (d.h., bis auf unwesentliche Zusätze und/oder Hilfsmittel) aus Superabsorber und Ketosäure oder besteht aus Superabsorber und Ketosäure. Der Superabsorber enthält wahlweise weitere Superabsorbern üblicherweise zugesetzte Zusätze oder Hilfsmittel, beispielsweise Entstaubungsmittel, Mittel zur Verbesserung der Fördereigenschaften oder Rieselfähigkeit des Superabsorbers.

Der in der erfindungsgemäßen Zusammensetzung enthaltene Superabsorber ist ein üblicher Superabsorber, der ein mehrfaches seines Eigengewichts an Wasser absorbieren und unter gewissem Druck zurückhalten kann. Im Allgemeinen weist er eine CRC ("Centrifuge Retention Capacity", Meßmethode siehe unten) von mindestens 5 g/g, vorzugsweise mindestens 10 g/g und in besonders bevorzugter Form mindestens 15 g/g auf. Vorzugsweise ist der Superabsorber ein vernetztes Polymer auf Basis teilneutralisierter Acrylsäure, und in besonders bevorzugter Form ist er oberflächennachvernetzt. Ein "Superabsorber" kann auch ein Gemisch stofflich verschiedener einzelner Superabsorber sein, es kommt hier weniger auf die stoffliche Zusammensetzung an als auf die superabsorbierenden Eigenschaften.

Verfahren zur Herstellung von Superabsorbern, auch oberflächennachvernetzten Superabsorbern, sind bekannt. Synthetische Superabsorber werden beispielsweise durch Polymerisation einer Monomerlösung, enthaltend
a) mindestens ein ethylenisch ungesättigtes, säuregruppentragendes Monomer,
b) mindestens einen Vernetzer,
c) wahlweise ein oder mehrere mit dem Monomeren a) copolymerisierbare ethylenisch und/oder allylisch ungesättigte Monomere und
d) wahlweise ein oder mehrere wasserlösliche Polymere, auf die die Monomere a), b) und gegebenenfalls c) zumindest teilweise aufgepfropft werden können,
erhalten.

Geeignete Monomere a) sind beispielsweise ethylenisch ungesättigte Carbonsäuren, wie Acrylsäure, Methacrylsäure, Maleinsäure, Fumarsäure und Itaconsäure, oder deren Derivate, wie Acrylamid, Methacrylamid, Acrylsäureester und Methacrylsäureester. Besonders bevorzugte Monomere sind Acrylsäure und Methacrylsäure. Ganz besonders bevorzugt ist Acrylsäure.

Die Monomere a), insbesondere Acrylsäure, enthalten vorzugsweise bis zu 0,025 Gew.-% eines Hydrochinonhalbethers. Bevorzugte Hydrochinonhalbether sind Hydrochinonmonomethylether (MEHQ) und/oder Tocopherole.

Unter Tocopherol werden Verbindungen der folgenden Formel verstanden 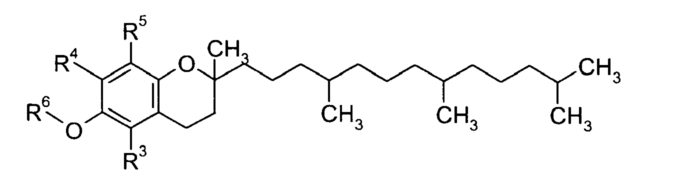 wobei R³ Wasserstoff oder Methyl, R⁴ Wasserstoff oder Methyl, R⁵ Wasserstoff oder Methyl und R⁴ Wasserstoff oder einen Säurerest mit 1 bis 20 Kohlenstoffatomen bedeutet.

Bevorzugte Reste für R⁶ sind Acetyl, Ascorbyl, Succinyl, Nicotinyl und andere physiologisch verträgliche Carbonsäuren. Die Carbonsäuren können Mono-, Di- oder Tricarbonsäuren sein.

Bevorzugt ist alpha-Tocopherol mit R³ = R⁴ = R⁵ = Methyl, insbesondere racemisches alpha-Tocopherol. R⁶ ist besonders bevorzugt Wasserstoff oder Acetyl. Insbesondere bevorzugt ist RRR-alpha-Tocopherol.

Die Monomerlösung enthält bevorzugt höchstens 130 Gew.-ppm, besonders bevorzugt höchstens 70 Gew.-ppm, bevorzugt mindesten 10 Gew.-ppm, besonders bevorzugt mindesten 30 Gew.-ppm, insbesondere um 50 Gew.-ppm, Hydrochinonhalbether, jeweils bezogen auf Acrylsäure, wobei Acrylsäuresalze als Acrylsäure mit berücksichtigt werden. Beispielsweise kann zur Herstellung der Monomerlösung eine Acrylsäure mit einem entsprechenden Gehalt an Hydrochinonhalbether verwendet werden.

Die Vernetzer b) sind Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Allylmethacrylat, Trimethylolpropantriacrylat, Triallylamin, Tetraallyloxyethan, wie in EP 530 438.A1 beschrieben, Di- und Triacrylate, wie in EP 547 847 A1, EP 559 476 A1, EP 632 068 A1, WO 93/21 237 A1, WO 03/104 299 A1, WO 03/104 300 A1, WO 03/104 301 A1 und DE 103 31 450 A1 beschrieben, gemischte Acrylate, die neben Acrylatgruppen weitere ethylenisch ungesättigte Gruppen enthalten, wie in DE 103 31 456 A1 und WO 04/013 064 A2 beschrieben, oder Vernetzermischungen, wie beispielsweise in DE 195 43 368 A1, DE 196 46 484 A1, WO 90/15 830 A1 und WO 02/032 962 A2 beschrieben.

Geeignete Vernetzer b) sind insbesondere N,N'-Methylenbisacrylamid und N,N'-Methylenbismethacrylamid, Ester ungesättigter Mono- oder Polycarbonsäuren von Polyolen, wie Diacrylat oder Triacrylat, beispielsweise Butandiol- oder Ethylenglykoldiacrylat bzw. -methacrylat sowie Trimethylolpropantriacrylat und Allylverbindungen, wie Allyl(meth)acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in EP 343 427 A2 beschrieben sind. Weiterhin geeignete Vernetzer b) sind Pentaerythritoldi-, Pentaerythritoltri- und Pentaerythritoltetraallylether, Polyethylenglykoldiallylether, Ethylenglykoldiallylether, Glycerindi- und Glycerintriallylether, Polyallylether auf Basis Sorbitol, sowie ethoxylierte Varianten davon. Im erfindungsgemäßen Verfahren einsetzbar sind Di(meth)acrylate von Polyethylenglykolen, wobei das eingesetzte Polyethylenglykol ein Molekulargewicht zwischen 300 und 1000 aufweist.

Besonders vorteilhafte Vernetzer b) sind jedoch Di- und Triacrylate des 3- bis 15-fach ethoxylierten Glycerins, des 3- bis 15-fach ethoxylierten Trimethylolpropans, des 3- bis 15-fach ethoxylierten Trimethylolethans, insbesondere Di- und Triacrylate des 2- bis 6- fach ethoxylierten Glycerins oder Trimethylolpropans, des 3-fach propoxylierten Glycerins oder Trimethylolpropans, sowie des 3-fach gemischt ethoxylierten oder propoxylierten Glycerins oder Trimethylolpropans, des 15-fach ethoxylierten Glycerins oder Trimethylolpropans, sowie des 40-fach ethoxylierten Glycerins, Trimethylolethans oder Trimethylolpropans.

Ganz besonders bevorzugte Vernetzer b) sind die mit Acrylsäure oder Methacrylsäure zu Di- oder Triacrylaten veresterten mehrfach ethoxylierten und/oder propoxylierten Glycerine wie sie beispielsweise in WO 03/104 301 A1 beschrieben sind. Besonders vorteilhaft sind Di- und/oder Triacrylate des 3- bis 10-fach ethoxylierten Glycerins. Ganz besonders bevorzugt sind Di- oder Triacrylate des 1- bis 5- fach ethoxylierten und/oder propoxylierten Glycerins. Am meisten bevorzugt sind die Triacrylate des 3-bis 5-fach ethoxylierten und/oder propoxylierten Glycerins. Diese zeichnen sich durch besonders niedrige Restgehalte (typischerweise unter 10 Gew.-ppm) im wasserabsorbierenden Polymer aus und die wässrigen Extrakte der damit hergestellten wasserabsorbierenden Polymere weisen eine fast unveränderte Oberflächenspannung (typischerweise mindestens 0,068 N/m) im Vergleich zu Wasser gleicher Temperatur auf.

Mit den Monomeren a) copolymerisierbare ethylenisch ungesättigte Monomere c) sind beispielsweise Acrylamid, Methacrylamid, Crotonsäureamid, Dimethylaminoethylmethacrylat, Dimethylaminoethylacrylat, Dimethylaminopropylacrylat, Diethylaminopropylacrylat, Dimethylaminobutylacrylat, Dimethylaminoethylmethacrylat, Diethylaminoethylmethacrylat, Dimethylaminoneopentylacrylat und Dimethylaminoneopentylmethacrylat.

Als wasserlösliche Polymere d) können Polyvinylalkohol, Polyvinylpyrrolidon, Stärke, Stärkederivate, Polyglykole, formal ganz oder teilweise aus Vinylaminmonomeren aufgebaute Polymere wie teilweise oder vollständig hydrolysiertes Polyvinylamid (sogenanntes "Polyvinylamin") oder Polyacrylsäuren, vorzugsweise Polyvinylalkohol und Stärke, eingesetzt werden.

Die Polymerisation wird wahlweise in Gegenwart üblicher Polymerisationsregler durchgeführt. Geeignete Polymerisationsregler sind beispielsweise Thioverbindungen, wie Thioglykolsäure, Mercaptoalkohole, z. B. 2-Mercaptoethanol, Mercaptopropanol und Mercaptobutanol, Dodecylmercaptan, Ameisensäure, Ammoniak und Amine, z. B. Ethanolamin, Diethanolamin, Triethanolamin, Triethylamin, Morpholin und Piperidin.

Die Monomere (a), (b) und gegebenenfalls (c) werden,wahlweise in Gegenwart der wasserlöslichen Polymere d), in 20 bis 80, vorzugsweise 20 bis 50, insbesondere 30 bis 45 Gew.-%iger wässriger Lösung in Gegenwart von Polymerisationsinitiatoren miteinander (co)polymerisiert. Als Polymerisationsinitiatoren können sämtliche unter den Polymerisationsbedingungen in Radikale zerfallende Verbindungen eingesetzt werden, z. B. Peroxide, Hydroperoxide, Wasserstoffperoxid, Persulfate, Azoverbindungen und die sogenannten Redoxinitiatoren. Bevorzugt ist der Einsatz von wasserlöslichen Initiatoren. In manchen Fällen ist es vorteilhaft, Mischungen verschiedener Polymerisationsinitiatoren zu verwenden, z. B. Mischungen aus Wasserstoffperoxid und Natrium- oder Kaliumperoxodisulfat. Mischungen aus Wasserstoffperoxid und Natriumperoxodisulfat können in jedem beliebigen Verhältnis verwendet werden. Geeignete organische Peroxide sind beispielsweise Acetylacetonperoxid, Methylethylketonperoxid, tert.-Butylhydroperoxid, Cumolhydroperoxid, tert.-Amylperpivalat, tert.-Butylperpivalat, tert.-Butylperneohexanoat, tert.-Butylperisobutyrat, tert.-Butylper-2-ethylhexanoat, tert.-Butylperisononanoat, tert.-Butylpermaleat, tert.-Butylperbenzoat, tert.-Butylper-3,5,5-trimethylhexanoat und tert.-Amylperneodekanoat. Weitere geeignete Polymerisationsinitiatoren sind Azostarter, z. B. 2,2'-Azobis-(2-amidinopropan)dihydrochlorid, 2,2'-Azobis-(N,N-dimethylen)isobutyramidin-dihydrochlorid, 2-(Carbamoylazo)isobutyronitril und 4,4'-Azobis-(4-cyanovaleriansäure). Die genannten Polymerisationsinitiatoren werden in üblichen Mengen eingesetzt, z. B. in Mengen von 0,01 bis 5, vorzugsweise 0,1 bis 2 Mol-%, bezogen auf die zu polymerisierenden Monomere.

Die Redoxinitiatoren enthalten als oxidierende Komponente mindestens eine der oben angegebenen Perverbindungen und eine reduzierende Komponente, beispielsweise Ascorbinsäure, Glukose, Sorbose, Ammonium- oder Alkalimetallhydrogensulfit, -sulfit, - thiosulfat, -hyposulfit, -pyrosulfit oder -sulfid, Metallsalze, wie Eisen-II-ionen oder Silberionen oder Natriumhydroxymethylsulfoxylat. Vorzugsweise verwendet man als reduzierende Komponente des Redoxinitiators Ascorbinsäure oder Natriumpyrosulfit. Bezogen auf die bei der Polymerisation eingesetzte Menge an Monomeren verwendet man 1 · 10⁻⁵ bis 1 Mol-% der reduzierenden Komponente des Redoxinitiators und 1 · 10⁻⁵ bis 5 Mol-% der oxidierenden Komponente. Anstelle der oxidierenden Komponente oder zusätzlich kann man auch einen oder mehrere wasserlösliche Azostarter verwenden.

Bevorzugt wird ein Redoxinitiator bestehend aus Wasserstoffperoxid, Natriumperoxodisulfat und Ascorbinsäure eingesetzt. Beispielsweise werden diese Komponenten in den Konzentrationen 1 · 10⁻² Mol-% Wasserstoffperoxid, 0,084 Mol-% Natriumperoxodisulfat und 2,5 · 10⁻³ Mol-% Ascorbinsäure bezogen auf die Monomere eingesetzt.

Die wässrige Monomerlösung kann den Initiator gelöst oder dispergiert enthalten. Die Initiatoren können dem Polymerisationsreaktor jedoch auch getrennt von der Monomerlösung zugeführt werden.

Die bevorzugten Polymerisationsinhibitoren benötigen für eine optimale Wirkung gelösten Sauerstoff. Daher können die Polymerisationsinhibitoren vor der Polymerisation durch Inertisierung, d.h. Durchströmen mit einem inerten Gas, vorzugsweise Stickstoff, von gelöstem Sauerstoff befreit werden. Dies geschieht mittels Inertgas, welches im Gleichstrom, Gegenstrom oder dazwischenliegenden Eintrittswinkeln eingeleitet werden kann. Eine gute Durchmischung kann beispielsweise mit Düsen, statischen oder dynamischen Mischern oder Blasensäulen erzielt werden. Vorzugsweise wird der Sauerstoffgehalt der Monomerlösung vor der Polymerisation auf weniger als 1 Gew.-ppm, besonders bevorzugt auf weniger als 0,5 Gew.-ppm, gesenkt. Die Monomerlösung wird wahlweise mit einem Inertgasstrom durch den Reaktor geführt.

Die Herstellung eines geeigneten Polymers sowie weitere geeignete hydrophile ethylenisch ungesättigte Monomere a) werden beispielsweise in DE 199 41 423 A1, EP 686 650 A1, WO 01/45 758 A1 und WO 03/104 300 A1 beschrieben.

Superabsorber werden üblicherweise durch Polymerisation einer wässrigen Monomerlösung und wahlweise einer anschließenden Zerkleinerung des Hydrogels erhalten.

Geeignete Herstellverfahren sind in der Literatur beschrieben. Superabsorber werden beispielsweise erhalten durch:
- Gelpolymerisation im Batchverfahren bzw. Rohrreaktor und anschließender Zerkleinerung im Fleischwolf, Extruder oder Kneter, wie beispielsweise in EP 445 619 A2 und DE 19 846 413 A1 beschrieben;
- Polymerisation im Kneter, wobei durch beispielsweise gegenläufige Rührwellen kontinuierlich zerkleinert wird, wie beispielsweise in WO 01/38 402 A1 beschrieben;
- Polymerisation auf dem Band und anschließende Zerkleinerung im Fleischwolf, Extruder oder Kneter wie beispielsweise in EP 955 086 A2, DE 38 25 366 A1 oder US 6 241 928 beschrieben;
- Emulsionspolymerisation, wobei bereits Perlpolymerisate relativ enger Gelgrößenverteilung anfallen, wie beispielsweise in EP 457 660 A1 beschrieben;
- In-situ Polymerisation einer Gewebeschicht, die zumeist im kontinuierlichen Betrieb zuvor mit wässriger Monomerlösung besprüht und anschließend einer Photopolymerisation unterworfen wurde, wie beispielsweise in WO 02/94 328 A2, WO 02/94 329 A1) beschrieben.

Bezüglich Details der Verfahrensdurchführung wird hiermit ausdrücklich auf die genannten Schriften verwiesen. Die Umsetzung wird vorzugsweise in einem Kneter oder auf einem Bandreaktor durchgeführt.

Der aus wirtschaftlichen Gründen bevorzugte und daher derzeit übliche Herstellungsprozess für Superabsorber ist die kontinuierliche Gelpolymerisation. Dabei wird zunächst eine Monomermischung hergestellt, indem zur Acrylsäurelösung in zeitlich und/oder räumlich getrennter Zugabefolge das Neutralisationsmittel, optionale Comonomere und/oder weitere Hilfsstoffe zugegeben werden, und die Mischung dann in den Reaktor überführt wird, oder bereits im Reaktor vorgelegt wird. Als letzte Zugabe erfolgt die Eindosierung des Initiatorsystems zum Start der Polymerisation. Im sich anschließenden kontinuierlichen Polymerisationsverfahren erfolgt die Reaktion zum Polymergel (d.h. dem im Lösungsmittel der Polymerisation - üblicherweise Wasser - zum Gel gequollenen Polymer), das im Falle einer gerührten Polymerisation bereits im Vorfeld zerkleinert wird. Das Polymergel wird anschließend getrocknet, falls erforderlich, auch gebrochen gemahlen und gesiebt und zur weiteren Oberflächenbehandlung transferiert.

Die Säuregruppen der erhaltenen Hydrogele sind üblicherweise teilweise neutralisiert, im Allgemeinen zu mindestens 25 Mol-%, vorzugsweise zu mindestens 27 Mol-% und in besonders bevorzugter Form mindestens 40 Mol-% sowie im Allgemeinen höchstens 85 Mol-%, vorzugsweise höchstens 80 Mol-% und in besonders bevorzugter Form höchstens 75 Mol-%, wozu die üblichen Neutralisationsmittel verwendet werden können, vorzugsweise Alkalimetallhydroxide, Alkalimetalloxide, Alkalimetallcarbonate oder Alkalimetallhydrogencarbonate sowie deren Mischungen. Statt Alkalimetallsalzen können auch Ammoniumsalze verwendet werden. Natrium und Kalium als Alkalimetalle besonders bevorzugt sind, ganz besonders bevorzugt jedoch Natriumhydroxid, Natriumcarbonat oder Natriumhydrogencarbonat sowie deren Mischungen. Üblicherweise wird die Neutralisation durch Einmischung des Neutralisationsmittels als wässrige Lösung oder bevorzugt auch als Feststoff erreicht. Beispielsweise kann Natriumhydroxid mit einem Wasseranteil deutlich unter 50 Gew.-% als wachsartige Masse mit einem Schmelzpunkt oberhalb 23°C vorliegen. In diesem Fall ist eine Dosierung als Stückgut oder Schmelze bei erhöhter Temperatur möglich.

Die Neutralisation kann nach der Polymerisation auf der Stufe des Hydrogels durchgeführt werden. Es ist aber auch möglich, die Neutralisation auf den gewünschten Neutralisationsgrad vollständig oder teilweise vor der Polymerisation durchzuführen. Bei teilweiser Neutralisation vor der Polymerisation werden im Allgemeinen mindestens 10 Mol-%, vorzugsweise mindestens 15 Mol-% sowie im Allgemeinen höchstens 40 Mol-%, vorzugsweise höchstens 30 Mol-% und in besonders bevorzugter Form höchstens 25 Mol-% der Säuregruppen in den eingesetzten Monomeren vor der Polymerisation neutralisiert, indem ein Teil des Neutralisationsmittels bereits der Monomerlösung zugesetzt wird. Der gewünschte Endneutralisationsgrad wird in diesem Fall erst gegen Ende oder nach der Polymerisation, vorzugsweise auf der Stufe des Hydrogels vor dessen Trocknung eingestellt. Die Monomerlösung wird durch Einmischen des Neutralisationsmittels neutralisiert. Das Hydrogel kann bei der Neutralisation mechanisch zerkleinert werden, beispielsweise mittels eines Fleischwolfes oder vergleichbaren Apparats zum Zerkleinern gelartiger Massen, wobei das Neutralisationsmittel aufgesprüht, übergestreut oder aufgegossen und dann sorgfältig untergemischt wird. Dazu kann die erhaltene Gelmasse noch mehrmals zur Homogenisierung gewolft werden.

In bevorzugter Form wird die Monomerlösung vor Polymerisation durch Zugabe des Neutralisationsmittels auf den gewünschten Endneutralisationsgrad eingestellt.

Die aus der Polymerisation erhaltenen Gele werden wahlweise einige Zeit, beispielsweise mindestens 30 Minuten, vorzugsweise mindestens 60 Minuten und in besonders bevorzugter Weise mindestens 90 Minuten sowie im Allgemeinen höchstens 12 Stunden, vorzugsweise höchstens 8 Stunden und in besonders bevorzugter Form höchstens 6 Stunden bei einer Temperatur von im Allgemeinen mindestens 50 °C und vorzugsweise mindestens 70 °C sowie im Allgemeinen höchstens 130 °C und vorzugsweise höchstens 100 °C gehalten, wodurch sich ihre Eigenschaften oft noch verbessern lassen.

Das neutralisierte Hydrogel wird dann mit einem Band- oder Walzentrockner getrocknet bis der Restfeuchtegehalt vorzugsweise unter 15 Gew.-%, insbesondere unter 10 Gew.-% liegt, wobei der Wassergehalt gemäß der von der EDANA (European Disposables and Nonwovens Association) empfohlenen Testmethode Nr. 430.2-02 "Moisture Content" bestimmt wird. Der trockene Superabsorber enthält folglich bis zu 15 Gew.-% Feuchtigkeit, vorzugsweise höchstens 10 Gew.-%. Entscheidend für die Einstufung als "trocken" ist insbesondere für Handhabung als Pulver (etwa zur pneumatischen Förderung, Abfüllung, Siebung oder sonstigen Verfahrensschritten aus der Feststoffverfahrenstechnik) ausreichende Rieselfähigkeit. Wahlweise kann zur Trocknung aber auch ein Wirbelbetttrockner oder ein beheizter Pflugscharmischer verwendet werden. Um besonders farblose Produkte zu erhalten, ist es vorteilhaft bei der Trocknung dieses Gels einen schnellen Abtransport des verdampfenden Wassers sicherzustellen. Dazu ist die Trocknertemperatur zu optimieren, die Luftzu- und -abführung muss kontrolliert erfolgen, und es ist in jedem Fall auf ausreichende Belüftung zu achten. Die Trocknung ist naturgemäß umso einfacher und das Produkt umso farbloser, je höher der Feststoffgehalt des Gels ist. Der Lösungsmittelanteil bei der Polymerisation wird daher so eingestellt, dass der Feststoffgehalt des Gels vor der Trocknung daher im Allgemeinen bei mindestens 20 Gew.-%, vorzugsweise bei mindestens 25 Gew.-% und in besonders bevorzugter Form bei mindestens 30 Gew.-% sowie im Allgemeinen bei höchstens 90 Gew.-%, vorzugsweise bei höchstens 85 Gew.-% und in besonders bevorzugter Form bei höchstens 80 Gew.-% liegt. Besonders vorteilhaft ist die Belüftung des Trockners mit Stickstoff oder einem anderen nicht-oxidierenden Inertgas. Wahlweise kann aber auch einfach nur der Partialdruck des Sauerstoffs während der Trocknung abgesenkt werden, um oxidative Vergilbungsvorgänge zu verhindern. Im Regelfall führt aber auch eine ausreichende Belüftung und Abführung des Wasserdampfes zu einem noch akzeptablen Produkt. Vorteilhaft hinsichtlich Farbe und Produktqualität ist in der Regel eine möglichst kurze Trocknungszeit.

Das getrocknete Hydrogel (das kein Gel mehr ist (auch wenn oft noch so genannt), sondern ein trockenes Polymer mit superabsorbierenden Eigenschaften, das unter den Begriff "Superabsorber" fällt) wird vorzugsweise gemahlen und gesiebt, wobei zur Mahlung üblicherweise Walzenstühle, Stiftmühlen, Hammermühlen, Schneidmühlen oder Schwingmühlen eingesetzt werden können. Die Partikelgröße des gesiebten, trockenen Hydrogels beträgt vorzugsweise unter 1000 µm, besonders bevorzugt unter 900 µm, ganz besonders bevorzugt unter 850 µm, und vorzugsweise über 80 µm, besonders bevorzugt über 90 µm, ganz besonders bevorzugt über 100 µm.

Ganz besonders bevorzugt ist eine Partikelgröße (Siebschnitt) von 106 bis 850 µm. Die Partikelgröße wird gemäß der von der EDANA (European Disposables and Nonwovens Association) empfohlenen Testmethode Nr. 420.2-02 "Particle size distribution" bestimmt.

Die so hergestellten trockenen superabsorbierenden Polymere werden üblicherweise als "Grundpolymere" bezeichnet und vorzugsweise anschließend oberflächennachvernetzt. Die Oberflächennachvernetzung kann in an sich bekannter Weise mit getrockneten, gemahlenen und abgesiebten Polymerpartikeln geschehen. Hierzu werden Verbindungen, die mit den funktionellen Gruppen des Grundpolymers unter Vernetzung reagieren können, meist in Form einer Lösung auf die Oberfläche der Grundpolymerpartikel aufgebracht. Geeignete Nachvernetzungsmittel sind beispielsweise:
- Di- oder Polyepoxide, etwa Di- oder Polyglycidylverbindungen wie Phosphonsäurediglycidylester, Ethylenglykoldiglycidylether oder Bischlorhydrinether von Polyalkylenglykolen,
- Alkoxysilylverbindungen,
- Polyaziridine, Aziridin-Einheiten enthaltende Verbindungen auf Basis von Polyethern oder substituierten Kohlenwasserstoffen, beispielsweise Bis-N-Aziridinomethan,
- Polyamine oder Polyamidoamine sowie deren Umsetzungsprodukte mit Epichlorhydrin,
- Polyole wie Ethylenglykol, 1,2-propandiol, 1,4-Butandiol, Glycerin, Methyltriglykol, Polyethylenglykole mit einem mittleren Molekulargewicht Mw von 200 - 10000, Di- und Polyglycerin, Pentaerythrit, Sorbit, die Oxethylate dieser Polyole sowie deren Ester mit Carbonsäuren oder der Kohlensäure wie Ethylencarbonat oder Propylencarbonat,
- Kohlensäurederivate wie Harnstoff, Thioharnstoff, Guanidin, Dicyandiamid, 2-Oxazolidinon und dessen Derivate, Bisoxazolin, Polyoxazoline, Di- und Polyisocyanate,
- Di- und Poly-N-methylolverbindungen wie beispielsweise Methylenbis(N-methylolmethacrylamid) oder Melamin-Forrrialdehyd-Harze,
- Verbindungen mit zwei oder mehr blockierten Isocyanat-Gruppen wie beispielsweise Trimethylhexamethylendiisocyanat blockiert mit 2,2,3,6-Tetramethylpiperidinon-4.

Bei Bedarf können saure Katalysatoren wie beispielsweise p-Toluolsulfonsäure, Phosphorsäure, Borsäure oder Ammoniumdihydrogenphosphat zugesetzt werden.

Besonders geeignete Nachvernetzungsmittel sind Di- oder Polyglycidylverbindungen wie Ethylenglykoldiglycidylether, die Umsetzungsprodukte von Polyamidoaminen mit Epichlorhydrin, 2-Oxazolidinon und N-Hydroxyethyl-2-oxazolidinon.

Die Oberflächennachvernetzung (oft auch nur "Nachvernetzung") wird üblicherweise so durchgeführt, dass eine Lösung des Oberflächennachvernetzers (oft auch nur "Nachvernetzer") auf das Hydrogel oder das trockene Grundpolymerpulver aufgesprüht wird.

Als Lösungsmittel für den Oberflächennachvernetzer wird ein übliches geeignetes Lösungsmittel verwendet, beispielsweise Wasser, Alkohole, DMF, DMSO sowie Mischungen davon. Besonders bevorzugt sind Wasser und Wasser/Alkohol-Mischungen wie zum Bespiel Wasser/Methanol, Wasser/1,2-Propandiol und Wasser/1,3-Propandiol.

Das Aufsprühen einer Lösung des Nachvernetzers wird vorzugsweise in Mischern mit bewegten Mischwerkzeugen, wie Schneckenmischern, Paddelmischern, Scheibenmischern, Pflugscharmischern und Schaufelmischern, durchgeführt werden. Besonders bevorzugt sind Vertikalmischer, ganz besonders bevorzugt sind Pflugscharmischer und Schaufelmischer. Geeignete und bekannte Mischer sind beispielsweise Lödige^{®}-, Bepex^{®}-, Nauta^{®}-, Processall^{®}- und Schugi^{®}-Mischer. Ganz besonders bevorzugt werden Hochgeschwindigkeitsmischer, beispielsweise vom Typ Schugi-Flexomix^{®} oder Turbolizer^{®}, eingesetzt.

Nach Aufsprühen der Vernetzer-Lösung kann wahlweise ein Temperaturbehandlungsschritt, im Wesentlichen zur Ausführung der Oberflächennachvernetzungsreaktion (dennoch meist nur als "Trocknung bezeichnet") nachfolgen, bevorzugt in einem nachgeschalteten beheizten Mischer ("Trockner"), bei einer Temperatur von im Allgemeinen mindestens 50 °C, vorzugsweise mindestens 80 °C und in besonders bevorzugter Form mindestens 90 °C sowie im Allgemeinen höchstens 250 °C, vorzugsweise höchstens 200 °C und in besonders bevorzugter Form höchstens 150 °C. Die mittlere Verweilzeit (also die gemittelte Verweilzeit der einzelnen Superabsorberpartikel) des zu behandelnden Superabsorbers im Trockner beträgt im Allgemeinen mindestens 1 Minute, vorzugsweise mindestens 3 Minuten und in besonders bevorzugter Form mindestens 5 Minuten sowie im Allgemeinen höchstens 6 Stunden, vorzugsweise höchstens 2 Stunden und in besonders bevorzugter Weise höchstens 1 Stunde. Dabei werden neben der eigentlichen Trocknung sowohl etwaige vorhandene Spaltprodukte als auch Lösungsmittelanteile entfernt. Die thermische Trocknung wird in üblichen Trocknern wie Hordentrocknern, Drehrohröfen oder beheizbaren Schnecken, vorzugsweise in Kontakttrocknern durchgeführt. Bevorzugt ist die Verwendung von Trocknern, in denen das Produkt bewegt wird, also beheizten Mischern, besonders bevorzugt Schaufeltrocknern, ganz besonders bevorzugt Scheibentrocknern. Geeignete Trockner sind beispielsweise Bepex^{®}-Trockner und Nara^{®}-Trockner. Überdies können auch Wirbelschichttrockner eingesetzt werden. Die Trocknung kann aber auch im Mischer selbst erfolgen, durch Beheizung des Mantels oder Einblasen eines vorgewärmten Gases wie Luft. Es kann aber auch beispielsweise eine azeotrope Destillation als Trocknungsverfahren benutzt werden. Die Vernetzungsreaktion kann sowohl vor als auch während der Trocknung stattfinden.

In einer besonders bevorzugten Ausführung der Erfindung wird zusätzlich die Hydrophilie der Partikeloberfläche der Grundpolymere durch Ausbildung von Komplexen modifiziert. Die Bildung der Komplexe auf der äußeren Schale der Partikel erfolgt durch Aufsprühen von Lösungen zwei- oder mehrwertiger Kationen, wobei die Kationen mit den Säuregruppen des Polymers unter Ausbildung von Komplexen reagieren können. Beispiele für zwei- oder mehrwertige Kationen sind formal ganz oder teilweise aus Vinylaminmonomeren aufgebaute Polymere wie teilweise oder vollständig hydrolysiertes Polyvinylamid (sogenanntes "Polyvinylamin"), dessen Amingruppen stets - auch bei sehr hohen pH-Werten - teilweise zu Ammoniumgruppen protoniert vorliegen oder Metallkationen wie Mg²⁺, Ca²⁺, Al³⁺, Sc³⁺, Ti⁴⁺, Mn²⁺, Fe^{2+/3+}, CO²⁺, Ni²⁺, Cu²⁺, Zn²⁺, Y³⁺, Zr⁴⁺, La³⁺, Ce⁴⁺, Hf⁴⁺, und Au³⁺. Bevorzugte Metall-Kationen sind Mg²⁺, Ca²⁺, Al³⁺, Ti⁴⁺, Zr⁴⁺ und La³⁺, und besonders bevorzugte Metall-Kationen sind Al³⁺, Ti⁴⁺ und Zr⁴⁺. Die Metall-Kationen können sowohl allein als auch im Gemisch untereinander eingesetzt werden. Von den genannten Metall-Kationen sind alle Metallsalze geeignet, die eine ausreichende Löslichkeit in dem zu verwendenden Lösungsmittel besitzen. Besonders geeignet sind Metallsalze mit schwach komplexierenden Anionen wie zum Beispiel Chlorid, Nitrat und Sulfat, Hydrogensulfat, Carbonat, Hydrogencarbonat, Nitrat, Phosphat, Hydrogenphosphat, Dihydrogenphosphat und Carboxylat, wie Acetat und Lactat. In besonders bevorzugter Form wird Aluminiumsulfat verwendet. Als Lösungsmittel für die Metallsalze können Wasser, Alkohole, DMF, DMSO sowie Mischungen dieser Komponenten eingesetzt werden. Besonders bevorzugt sind Wasser und Wasser/Alkohol-Mischungen wie zum Bespiel Wasser/Methanol, Wasser/1,2-Propandiol und Wasser/1,3-Propandiol.

Die Behandlung des Grundpolymeren mit Lösung eines zwei- oder mehrwertigen Kations erfolgt in gleicher Weise wie die mit Oberflächennachvernetzer, einschließlich des wahlweisen Trocknungsschritts. Oberflächennachvernetzer und polyvalentes Kation können in einer gemeinsamen Lösung oder als getrennte Lösungen aufgesprüht werden. Das Aufsprühen der Metallsalz-Lösung auf die Superabsorberpartikel kann sowohl vor als auch nach der Oberflächennachvernetzung erfolgen. In einem besonders bevorzugten Verfahren erfolgt die Aufsprühung der Metallsalz-Lösung im gleichen Schritt mit dem Aufsprühen der Vernetzer-Lösung, wobei beide Lösungen getrennt nacheinander oder gleichzeitig über zwei Düsen aufgesprüht werden, oder Vernetzer- und Metallsalz-Lösung vereint über eine Düse aufgesprüht werden können.

Sofern im Anschluss an die Oberflächennachvernetzung und/oder Behandlung mit Komplexbildner ein Trocknungsschritt durchgeführt wird, ist es vorteilhaft, aber nicht unbedingt notwendig, das Produkt nach der Trocknung zu kühlen. Die Kühlung kann kontinuierlich oder diskontinuierlich erfolgen, bequemerweise wird das Produkt dazu kontinuierlich in einen dem Trockner nachgeschalteten Kühler gefördert. Dazu kann jeder zur Abfuhr von Wärme aus pulverförmigen Feststoffen bekannte Apparat verwendet werden, insbesondere jede oben als Trocknungsapparat erwähnte Vorrichtung, sofern sie nicht mit einem Heizmedium, sondern mit einem Kühlmedium wie etwa mit Kühlwasser beaufschlagt wird, so dass über die Wände und je nach Konstruktion auch über die Rührorgane oder sonstige Wärmeaustauschflächen keine Wärme in den Superabsorber eingetragen, sondern daraus abgeführt wird. Bevorzugt ist die Verwendung von Kühlern, in denen das Produkt bewegt wird, also gekühlten Mischern. beispielsweise Schaufelkühlern, Scheibenkühlern oder Paddelkühler wie etwa in Nara^{®}- oder Bepex^{®}-Kühlern. Der Superabsorber kann auch in der Wirbelschicht durch Einblasen eines gekühlten Gases wie kalter Luft gekühlt werden. Die Bedingungen der Kühlung werden so eingestellt, dass ein Superabsorber mit der für die Weiterverarbeitung gewünschten Temperatur erhalten wird. Typischerweise wird eine mittlere Verweilzeit im Kühler von im Allgemeinen mindestens 1 Minute, vorzugsweise mindestens 3 Minuten und in besonders bevorzugter Form mindestens 5 Minuten sowie im Allgemeinen höchstens 6 Stunden, vorzugsweise höchstens 2 Stunden und in besonders bevorzugter Weise höchstens 1 Stunde eingestellt und die Kühlleistung so bemessen, dass das erhaltene Produkt eine Temperatur von im Allgemeinen mindestens 0 °C, vorzugsweise mindestens 10 °C und in besonders bevorzugter Form mindestens 20 °C sowie im Allgemeinen höchstens 100 °C, vorzugsweise höchstens 80 °C und in besonders bevorzugter Form höchstens 60 °C aufweist.

Optional kann noch eine weitere Modifizierung der Superabsorber durch Zumischung feinteiliger anorganischer Feststoffe, wie zum Beispiel Siliziumdioxid, Aluminiumoxid, Titandioxid und Eisen(II)-oxid erfolgen, wodurch die Effekte der Oberflächennachbehandlung noch weiter verstärkt werden. Besonders bevorzugt ist die Zumischung von hydrophilem Siliziumdioxid oder von Aluminiumoxid mit einer mittleren Größe der Primärteilchen von 4 bis 50 nm und einer spezifischen Oberfläche von 50 - 450 m²/g. Die Zumischung feinteiliger anorganischer Feststoffe erfolgt bevorzugt nach der Oberflächenmodifizierung durch Vernetzung/Komplexbildung, kann aber auch vor oder während diesen Oberflächenmodifizierungen durchgeführt werden.

Wahlweise wird Superabsorber mit weiteren üblichen Zusätzen und Hilfsstoffen versehen, die Lagerungs- oder Handhabungseigenschaften beeinflussen. Beispiele dafür sind Einfärbungen, opake Zusätze, um die Sichtbarkeit gequollenen Gels zu verbessern, was in manchen Anwendungen wünschenswert ist, Zusätze zur Verbesserung des Fließverhaltens des Pulvers, Tenside oder Ähnliches. Oft wird dem Superabsorber Entstaubungs- oder Staubbindemittel zugegeben. Entstaubungs- oder Staubbindemittel sind bekannt, beispielsweise werden Polyetherglykole wie Polyethylenglykol mit einem Molekulargewicht von 400 bis 20 000 g/Mol, Polyole wie Glycerin, Sorbit, Neopentylglykol oder Trimethylolpropan, die wahlweise auch 7 bis 20-fach ethoxyliert sind, verwendet. Auch ein endlicher Wassergehalt des Superabsorbers kann durch Wasserzugabe eingestellt werden, sofern gewünscht.

Die Feststoffe, Zusätze und Hilfsstoffe können jeweils in separaten Verfahrensschritten zugegeben werden, meist ist jedoch die bequemste Methode, sie dem Superabsorber im Kühler zuzugeben, etwa durch Aufsprühen einer Lösung oder Zugabe in feinteiliger fester oder in flüssiger Form.

Der oberflächennachvernetzte Superabsorber wird wahlweise in üblicher Weise gemahlen und/oder gesiebt. Mahlung ist hier typischerweise nicht erforderlich, meist ist aber zur Einstellung der gewünschten Partikelgrößenverteilung des Produkts das Absieben von gebildeten Agglomeraten oder Feinkorn angebracht. Agglomerate und Feinkorn werden entweder verworfen oder vorzugsweise in bekannter Weise und an geeigneter Stelle in das Verfahren zurückgeführt; Agglomerate nach Zerkleinerung. Die Partikelgröße der Superabsorberpartikel beträgt vorzugsweise höchstens 1000 µm, besonders bevorzugt höchstens 900 µm, ganz besonders bevorzugt höchstens 850 µm, und vorzugsweise mindestens 80 µm, besonders bevorzugt mindestens 90 µm, ganz besonders bevorzugt mindestens 100 µm. Typische Siebschnitte sind beispielsweise 106 bis 850 µm oder 150 bis 850 µm.

Die erfindungsgemäße Zusammensetzung wird hergestellt durch Zugabe mindestens einer Ketosäure zu einem Superabsorber. Dazu führt man vor während oder nach der Herstellung des Superabsorbers mindestens einen der folgenden Schritte durch:
i) Mischen mindestens einer Ketosäure mit einem Superabsorber;
ii) gemeinsames Mahlen mindestens einer Ketosäure mit einem Superabsorber;
iii) Aufsprühen mindestens einer Ketosäure, wahlweise in einem Lösungsmittel gelöst, auf einen Superabsorber und/oder
iv) bei durch Polymerisation mindestens eines Monomers hergestellten Superabsorbern, Zugabe mindestens einer Ketosäure zur Monomerlösung oder zur Reaktionsmischung während der Polymerisation.

Beim Mischen mindestens einer Ketosäure mit einem Superabsorber wird die Ketosäure oder das Ketosäuregemisch mit dem Superabsorber (der auch ein Grundpolymer vor der Nachvernetzung sein kann) auf jede beliebige Art gemischt. Verfahren und Apparate zum Mischen sind bekannt. Der Superabsorber kann beispielsweise in den oben bei der Nachvernetzung genannten Mischern und Trocknern mit Ketosäure vermischt werden, bequemerweise während der Nachvernetzung. Nachträgliches Einmischen der Ketosäure(n) ist jedoch ebenfalls möglich, wenn diese in ausreichend fester Form vorliegen, bei weicheren, etwa wachsartigen Substanzen kann das Vermischen auch unter Kühlung erfolgen.

Beim gemeinsamen Mahlen mindestens einer Ketosäure und eines Superabsorbers unterliegt die Art des Mahlens keiner Beschränkung. Geeignete Apparate sind bekannt und wurden bereits oben bei der Zerkleinerung des Grundpolymers beschrieben. Bequemerweise wird die Ketosäure oder das Ketosäuregemisch während eines Mahlschritts bei der Herstellung des Superabsorbers zugegeben. Bei weicheren, etwa wachsartigen Substanzen kann das gemeinsame Mahlen auch unter Kühlung erfolgen Beim Aufsprühen der Ketosäure oder Ketosäuren unterliegt die Art des Aufsprühens ebenfalls keiner Beschränkung. Die Ketosäure oder das Ketosäuregemisch kann als Schmelze (diese vorzugsweise als feiner Sprühnebel) oder vorzugsweise in Form einer Lösung aufgesprüht werden, beispielsweise und vorzugsweise während der Nachvernetzung des Grundpolymers in den dort genannten Mischern, in denen der Oberflächennachvernetzer und/oder die Metallsalzlösung aufgesprüht wird. Als Lösungsmittel für die Ketosäure oder das Ketosäuregemisch wird ein geeignetes Lösungsmittel verwendet, beispielsweise Wasser, Wasser/Aceton-Gemische, Wasser/Propylenglykol- oder Wasser/1,3-Propandiol-Gemische sowie die bei der Nachvernetzung und Metallsalzbehandlung genannten Lösungsmittel und Lösungsmittelgemische. Die Konzentration an Ketosäure in der Lösung beträgt im Allgemeinen mindestens 0,5 Gew.-%, vorzugsweise mindestens 1 Gew.-% und in besonders bevorzugter Form mindestens 2 Gew.-% sowie im Allgemeinen höchstens 30 Gew.-%, vorzugsweise höchstens 20 Gew.-% und in besonders bevorzugter Form höchstens 10 Gew.-%. Bequemerweise wird die Ketosäure gemeinsam mit Oberflächennachvernetzungsmittel und gegebenenfalls Metallsalz im Oberflächennachvernetzungsschritt aufgebracht. Meist werden die Lösungen über getrennte Düsen aufgesprüht, sofern Nachvernetzer, Metallsalz und Ketosäure keine unerwünschten Reaktionen miteinander eingehen, können sie allerdings auch in Form einer gemeinsamen Lösung aufgesprüht werden.

In einer weiteren Ausführungsform wird mittels der oben beschriebenen Verfahren eine erfindungsgemäße Zusammensetzung hergestellt, die einen höheren Anteil der mindestens einen Ketosäure aufweist, im Allgemeinen mindestes 1 Gew.-%, vorzugsweise mindestens 5 Gew.-% und in besonders bevorzugter Form mindestens 10 Gew.-% sowie im Allgemeinen höchstens 50 Gew.-%, vorzugsweise höchstens 40 Gew.-% und in besonders bevorzugter Form höchstens 30 Gew.-%. Die so erhaltene Zusammensetzung kann dann durch Vermischen in üblichen Apparaten mit weiterem Superabsorber auf den gewünschten Endgehalt an Ketosäure verdünnt werden.

Die erfindungsgemäße Zusammensetzung wird, falls zur Einstellung der gewünschten Partikelgrößenverteilung erforderlich, nach einem nachträglichen Aufbringen von oder Vermischen mit Ketosäure nochmals gesiebt.

Ferner wurden Hygieneartikel gefunden, die den erfindungsgemäßen Superabsorber enthalten. Erfindungsgemäße Hygieneartikel sind beispielsweise solche, die zur Anwendung bei leichter oder schwerer Inkontinenz vorgesehen sind, wie etwa Einlagen für schwere oder leichte Inkontinenz, Inkontinenzhosen, daneben Windeln, sogenannte "training pants" für Babys und Kleinkinder oder auch Damenhygieneartikel wie Einlagen, Damenbinden oder Tampons. Derartige Hygieneartikel sind bekannt. Die erfindungsgemäßen Hygieneartikel unterscheiden sich von bekannten dadurch, dass sie den erfindungsgemäßen Superabsorber enthalten. Es wurde außerdem ein Verfahren zur Herstellung von Hygieneartikeln gefunden, das dadurch gekennzeichnet ist, dass man bei der Herstellung des betreffenden Hygieneartikels mindestens einen erfindungsgemäßen Superabsorber einsetzt. Im übrigen sind Verfahren zur Herstellung von Hygieneartikeln unter Einsatz von Superabsorber bekannt.

Die vorliegende Erfindung betrifft ferner die Verwendung der erfindungsgemäßen Zusammensetzung in Trainingsunterwäsche für Kinder (sogenannte "training pants", d.h. Wäsche zur Sauberkeitserziehung), Schuheinlagen und anderen Hygieneartikeln zur Absorption von Körperflüssigkeiten. Die erfindungsgemäße Zusammensetzung kann außerdem in anderen Gebieten der Technik eingesetzt werden, bei denen Flüssigkeiten, insbesondere Wasser oder wässrige Lösungen absorbiert werden. Diese Gebiete sind beispielsweise Lagerung, Verpackung, Transport (als Bestandteile von Verpackungsmaterial für wasser- oder feuchtigkeitsempfindliche Artikel, etwa zum Blumentransport, auch als Schutz gegen mechanische Einwirkungen); Tierhygiene (in Katzenstreu); Lebensmittelverpackung (Transport von Fisch, Frischfleisch; Absorption von Wasser, Blut in Frischfisch- oder -fleischverpackungen); Medizin (Wundpflaster, wasserabsorbierendes Material für Brandverbände oder für andere nässende Wunden), Kosmetik (Trägermaterial für Pharmachemikalien und Medikamente, Rheumapflaster, Ultraschallgel, Kühlgel, Kosmetikverdicker, Sonnenschutz); Verdicker für Öl/Wasser bzw. Wasser/Öl-Emulsionen; Textilien (Feuchtigkeitsregulation in Textilien, Schuheinlagen, zur Verdampfungskühlung, etwa in Schutzkleidung, Handschuhen, Stirnbändern); chemisch-technische Anwendungen (als Katalysator für org. Reaktionen, zur Immobilisierung großer funktioneller Moleküle wie Enzymen, als Adhäsionsmittel bei Agglomerationen, Wärmespeicher, Filtrationshilfsmittel, hydrophile Komponente in Polymerlaminaten, Dispergiermittel, Verflüssiger); als Hilfsmittel beim Pulverspritzguss, im Bau- und Konstruktionswesen (Installation, in lehmbasierenden Putzen, als vibrationshemmendes Medium, Hilfsmittel bei Tunnelgrabungen in wasserreichem Untergrund, Kabelummantelung); Wasserbehandlung, Abfallbehandlung, Wasserabtrennung (Enteisungsmittel, wiederverwendbare Sandsäcke); Reinigung; Agrarindustrie (Bewässerung, Rückhaltung von Schmelzwasser und Tauniederschläge, Kompostierungszusatz, Schutz der Wälder vor Pilz-/Insektenbefall, verzögerte Freisetzung von Wirkstoffen an Pflanzen); zur Brandbekämpfung oder zum Brandschutz; Coextrusionsmittel in thermoplastischen Polymeren (z. B. zur Hydrophilierung von Mehrschichtfolien); Herstellung von Folien und thermoplastischen Formkörpern, die Wasser absorbieren können (z.B. Regen- und Tauwasser speichernde Folien für die Landwirtschaft; Superabsorber enthaltende Folien zum Frischhalten von Obst und Gemüse, die in feuchte Folien verpackt werden; Superabsorber-Polystyrol Coextrudate, beispielsweise für Lebensmittelverpackungen wie Fleisch, Fisch, Geflügel, Obst und Gemüse); oder als Trägersubstanz in Wirkstoffformulierungen (Pharma, Pflanzenschutz).

### Testmethoden

### Zentrifugenretentionskapazität ("CRC", "Centrifuge Retention Capacity"):

Die Centrifuge Retention Capacity (CRC) wird gemäß der von der EDANA (European Disposables and Nonwovens Association, Avenue Eugène Plasky 157, 1030 Brüssel, Belgien) empfohlenen und von dort erhältlichen Testmethode Nr. 441.2-02 "Centrifuge Retention Capacity" bestimmt.

### Bestimmung der Geruchsinhibierung:

Zur Bewertung des geruchsinhibierenden Effekts der erfindungsgemäßen Zusammensetzungen wird die Inhibierung der Aktivität von Urease bei der Bildung von Ammoniak aus Harnstoff bestimmt. Die Bestimmung erfolgt bei Raumtemperatur. 2 g der zu prüfenden Substanz (Superabsorber, erfindungsgemäße Zusammensetzung) werden in einen 100 ml Erlenmeyer-Kolben eingewogen. 30 mg feste Urease (aus Schwertbohnen; lyophilisiert 5 U/mg zur Harnstoffbestimmung im Serum, Merck, Artikelnr. 4194753) wird in einem 100ml-Becherglas einwogen. Zur Urease werden 50 ml 0,9%ige Kochsalzlösung, in der Harnstoff gelöst ist (8,56g/L) zugegeben. Der gesamte Inhalt des Becherglases wird umgehend zur Probe in den Erlenmeyer-Kolben gegossen, ein Diffusionsröhrchen (Dräger-Röhrchen, Ammoniak 20 / a-D, 20 - 1500 ppm*h, Bestellnr. 8101301) wird wie vorgesehen aufgebrochen, in einen auf den Erlenmeyer-Kolben passenden Gummistopfen gesteckt und der Erlenmeyer-Kolben mit dem Stopfen so verschlossen, dass das offene Ende des Diffusionsröhrchens nach innen weist. Über insgesamt 6 Stunden wird alle 30 Minuten der vom Diffusionsröhrchen angezeigte Wert abgelesen und notiert. Die Messung wird jeweils als Doppelbestimmung durchgeführt, als Ergebnis wird der auf die nächste ganze Zahl gerundete Mittelwert der beiden Ablesungen verwendet.

### Beispiele

Der in den folgenden Beispielen verwendete, oberflächennachvernetzte Superabsorber wurde nach dem Verfahren von Beispiel 1 von WO 01/038 402 A1 hergestellt, jedoch mit einem Neutralisationsgrad von 72 Mol-% (statt 77 Mol-%) und in einem Siebschnitt von 106 bis 850 µm (statt <800 µm). Gewichtsprozentangaben für die eingesetzten Ketosäuren beziehen sich auf die Menge an eingesetztem Superabsorber.

### Vergleichsbeispiel V1

Superabsorber wurde ohne Ketosäurezusatz getestet.

### Vergleichsbeispiel V2

Superabsorber wurde in einer handelsüblichen Küchenmaschine mit 1,5 Gew.-% Glyoxylsäure vermischt, indem die entsprechende Menge 50 Gew.-%iger wässriger Glyoxylsäurelösung unter Rühren aufgesprüht wurde.

### Vergleichsbeispiel V3

Superabsorber wurde in einer handelsüblichen Küchenmaschine mit 3,0 Gew.-% Glyoxylsäure vermischt, indem die entsprechende Menge 50 Gew.-%iger wässriger Glyoxylsäurelösung unter Rühren aufgesprüht wurde.

### Vergleichsbeispiel V4

Superabsorber wurde in einer handelsüblichen Küchenmaschine mit 6,0 Gew.-% Glyoxylsäure vermischt, indem die entsprechende Menge 50 Gew.-%iger wässriger Glyoxylsäurelösung unter Rühren aufgesprüht wurde.

### Beispiel 1

Superabsorber wurde mit 0,5 Gew.-% fein gemahlener 2-Keto-L-Gulonsäure durch rund 20-minütiges Taumeln in einem Taumelmischer vermischt.

### Beispiel 2

Superabsorber wurde mit 1,0 Gew.-% fein gemahlener 2-Keto-L-Gulonsäure durch rund 20-minütiges Taumeln in einem Taumelmischer vermischt.

### Beispiel 3

Superabsorber wurde mit 3,0 Gew.-% fein gemahlener 2-Keto-L-Gulonsäure durch rund 20-minütiges Taumeln in einem Taumelmischer vermischt.

### Beispiel 4

Superabsorber wurde mit 0,5 Gew.-% fein gemahlener 2-Oxoglutarsäure durch rund 20-minütiges Taumeln in einem Taumelmischer vermischt.

### Beispiel 5

Superabsorber wurde mit 1,0 Gew.-% fein gemahlener 2-Oxoglutarsäure durch rund 20-minütiges Taumeln in einem Taumelmischer vermischt.

### Beispiel 6

Superabsorber wurde mit 3,0 Gew.-% fein gemahlener 2-Oxoglutarsäure durch rund 20-minütiges Taumeln in einem Taumelmischer vermischt.

Die CRC und der geruchsinhibierende Effekt der in den Beispielen erhaltenen Superabsorber und Zusammensetzungen wurde wie oben beschrieben bestimmt. Die Ergebnisse sind in Tabelle 1 aufgeführt.

Der Vergleich der Beispiele mit den Vergleichsbeispielen zeigt, dass der bekannte Geruchsinhibitor Glyoxylsäure einen geruchsinhibierenden Effekt zeigt, die Ketosäuren jedoch in weit geringerer Konzentration schon einen weit stärkeren geruchsinhibierenden Effekt aufweisen. Die Absorptionsleistung des Superabsorbers wird durch den Säurezusatz nur unwesentlich beeinträchtigt.

**Tabelle 1**

| | Beispiel Nr. | | | | | | | | | |
|---|---|---|---|---|---|---|---|---|---|---|
| | V1 | V2 | V3 | V4 | 1 | 2 | 3 | 4 | 5 | 6 |
| | - | Glyoxylsäure, [Gew.%] | | | 2-Keto-L-Gulonsäure, in Gew.-% | | | 2-Oxoglutarsäure, in Gew.-% | | |
| | - | 1,5 | 3,0 | 6,0 | 0,5 | 1,0 | 3,0 | 0,5 | 3,0 | 1,0 |
| | | | | | | | | | | |
| CRC [g/g] | 30,0 | 28,5 | 28,3 | 27,3 | 29,6 | 28,9 | 28,4 | 28,9 | 27,7 | 29,8 |
| | | | | | | | | | | |
| Zeit [h] | Bestimmung der Geruchsinhibierung | | | | | | | | | |
| 0,5 | 5 | 35 | 45 | 48 | 10 | 3 | 5 | 3 | 5 | 3 |
| 1 | 16,5 | 55 | 78 | 85 | 20 | 3 | 5 | 15 | 13 | 3 |
| 1,5 | 45 | 83 | 100 | 100 | 55 | 5 | 8 | 40 | 15 | 3 |
| 2 | 100 | 100 | 130 | 115 | 100 | 8 | 8 | 85 | 25 | 3 |
| 2,5 | 185 | 125 | 145 | 135 | 160 | 10 | 8 | 170 | 28 | 3 |
| 3 | 260 | 160 | 180 | 150 | 205 | 13 | 8 | 255 | 30 | 3 |
| 3,5 | 350 | 215 | 225 | 180 | 270 | 23 | 8 | 310 | 33 | 3 |
| 4 | 440 | 250 | 275 | 200 | 325 | 30 | 8 | 410 | 33 | 3 |
| 4,5 | 525 | 345 | 350 | 205 | 405 | 55 | 8 | 495 | 33 | 3 |
| 5 | 625 | 400 | 400 | 235 | 425 | 75 | 8 | 590 | 33 | 3 |
| 5,5 | 740 | 450 | 450 | 270 | 500 | 105 | 8 | 675 | 33 | 3 |
| 6 | 850 | 550 | 575 | 300 | 575 | 138 | 8 | 800 | 33 | 3 |

### Beispiele 7-12

Jeweils 1,0 g Superabsorber nach Beispielen 2 (1,0 Gew.-% Keto-L-Gulonsäure) und 3 (3,0 Gew.-% Keto-L-Gulonsäure) wurden in 100 ml physiologischer Kochsalzlösung (0,9 Gew.-%ige NaCl-Lösung in Wasser) suspendiert und mit jeweils 0,1 ml einer Keimsuspension versetzt. Nach Inkubationszeiten bei Raumtemperatur von 10 Minuten, 4 Stunden und 8 Stunden wurden Proben entnommen und die Anzahl koloniebildender Einheiten (üblicherweise englisch als "colony foming units", kurz"cfu" bezeichnet) bestimmt. Verwendet wurden die folgenden drei Keimsuspensionen mit den angegebenen Ausgangskonzentrationen:

| | |
|---|---|
| Escherichia coli | 1,6 · 10⁶ cfu/ml |
| Staphylococcus aureus | 2,2 · 10⁶ cfu/ml |
| Proteus mirabilis | 1,4 · 10⁶ cfu/ml |

Die Ergebnisse sind in der folgenden Tabelle 2 zusammengefasst

**Tabelle 2**

| Beispiel | Keim | Superabsorber | Keimanzahl [in 10⁵ cfu/g] nach | | |
|---|---|---|---|---|---|
| | | | 10 min | 4 h | 8h |
| 7 | Escherichia coli | Bsp. 2 | 1,3 | 2,4 | 2,9 |
| 8 | Staphylococcus aureus | Bsp.2 | 1,7 | 2,5 | 2,7 |
| 9 | Proteus mirabilis | Bsp.2 | 1,9 | 2,9 | 3,0 |
| 10 | Escherichia coli | Bsp.3 | 2,3 | 1,2 | 2,4 |
| 11 | Staphylococcus aureus | Bsp.3 | 1,5 | 1,9 | 1,4 |
| 12 | Proteus mirabilis | Bsp.3 | 1,8 | 1,7 | 3,1 |

Die Beispiele 7-12 zeigen, dass der Keto-L-Gulonsäure enthaltende Superabsorber keinen signifikanten bakteriziden Effekt hat.

## Patentansprüche

1. Zusammensetzung, die Superabsorber und 2-Keto-L-Gulonsäure und/oder 2-Keto-Glutarsäure enthält.

2. Zusammensetzung nach Anspruch 1, **dadurch gekennzeichnet, dass** die Menge der 2-Keto-L-Gulonsäure und/oder 2-Keto-Glutarsäure 0,005 bis 15 Gew.-%, bezogen auf die Superabsorbermenge ist.

3. Zusammensetzung nach einem der Ansprüche 1 bis 2, **dadurch gekennzeichnet, dass** der Superabsorber ein vernetztes Polymer auf Basis teilneutralisierter Acrylsäure ist.

4. Zusammensetzung nach einem der Ansprüche 1 bis 3, **dadurch gekennzeichnet, dass** der Superabsorber oberflächennachvernetzt ist.

5. Verfahren zur Herstellung einer in den Ansprüchen 1 bis 4 definierten Zusammensetzung, **dadurch gekennzeichnet, dass** man bei der Herstellung eines Superabsorbers mindestens einen der folgenden Schritte durchführt:
i) Zumischen von 2-Keto-L-Gulonsäure und/oder 2-Keto-Glutarsäure;
ii) gemeinsames Mahlen des Superabsorbers mit 2-Keto-L-Gulonsäure und/oder 2-Keto-Glutarsäure;
iii) Aufsprühen von 2-Keto-L-Gulonsäure und/oder 2-Keto-Glutarsäure, wahlweise in einem Lösungsmittel gelöst, auf den Superabsorber und/oder
iv) bei durch Polymerisation mindestens eines Monomers hergestellten Superabsorbern, Zugabe von 2-Keto-L-Gulonsäure und/oder 2-Keto-Glutarsäure zur Monomerlösung oder zur Reaktionsmischung während der Polymerisation.

6. Hygieneartikel, enthaltend eine in den Ansprüchen 1 bis 4 definierte Zusammensetzung.

7. Hygieneartikel nach Anspruch 6, **dadurch gekennzeichnet, dass** der Hygieneartikel zur Anwendung bei schwerer und/oder leichter Inkontinenz vorgesehen ist.

8. Verfahren zur Herstellung von Hygieneartikeln, **dadurch gekennzeichnet, dass** bei der Herstellung mindestens eine in den Ansprüchen 1 bis 4 definierte Zusammensetzung eingesetzt wird.

## Claims

1. A composition comprising superabsorbent and 2-keto-L-gulonic acid and/or 2-ketoglutaric acid.

2. The composition according to claim 1 wherein the amount of 2-keto-L-gulonic acid and/or 2-ketoglutaric acid is in the range from 0.005% to 15% by weight, based on the amount of superabsorbent.

3. The composition according to either of claims 1 and 2 wherein the superabsorbent is a crosslinked polymer based on partially neutralized acrylic acid.

4. The composition according to any one of claims 1 to 3 wherein the superabsorbent is surface postcrosslinked.

5. A process for producing a composition defined in claims 1 to 4, which comprises producing a superabsorbent by performing at least one of the following steps:
i) admixing 2-keto-L-gulonic acid and/or 2-ketoglutaric acid;
ii) conjointly grinding the superabsorbent with 2-keto-L-gulonic acid and/or 2-ketoglutaric acid;
iii) spraying the superabsorbent with 2-keto-L-gulonic acid and/or 2-ketoglutaric acid, optionally dissolved in a solvent; and/or
iv) in the case of superabsorbents being produced by addition polymerization of at least one monomer, adding 2-keto-L-gulonic acid and/or 2-ketoglutaric acid to the monomer solution or to the reaction mixture during the addition polymerization.

6. A hygiene article comprising a composition defined in claims 1 to 4.

7. The hygiene article according to claim 6 for use in severe and/or mild incontinence.

8. A process for producing a hygiene article, which comprises utilizing at least one composition defined in claims 1 to 4.

## Revendications

1. Composition qui contient un superabsorbant et de l'acide 2-céto-L-gulonique et/ou de l'acide 2-céto-glutarique.

2. Composition selon la revendication 1, **caractérisée en ce que** la quantité d'acide 2-céto-L-gulonique et/ou d'acide 2-céto-glutarique est de 0,005 à 15% en poids, par rapport à la quantité de superabsorbant.

3. Composition selon l'une quelconque des revendications 1 à 2, **caractérisée en ce que** le superabsorbent est un polymère réticulé à base d'acide acrylique partiellement neutralisé.

4. Composition selon l'une quelconque des revendications 1 à 3, **caractérisée en ce que** le superabsorbant est réticulé en surface.

5. Procédé pour la préparation d'une composition définie dans les revendications 1 à 4, **caractérisé en ce qu'**on réalise au moins une des étapes suivantes lors de la préparation d'un superabsorbant :
i) addition et mélange d'acide 2-céto-L-gulonique et/ou d'acide 2-céto-glutarique ;
ii) broyage commun du superabsorbant avec de l'acide 2-céto-L-gulonique et/ou de l'acide 2-céto-glutarique ;
iii) pulvérisation d'acide 2-céto-L-gulonique et/ou d'acide 2-céto-glutarique, au choix dissous dans un solvant, sur le superabsorbant et/ou
iv) dans le cas de superabsorbants préparés par polymérisation d'au moins un monomère, addition d'acide 2-céto-L-gulonique et/ou d'acide 2-céto-glutarique à la solution de monomère ou au mélange réactionnel pendant la polymérisation.

6. Article hygiénique, contenant une composition définie dans les revendications 1 à 4.

7. Article hygiénique selon la revendication 6, **caractérisé en ce que** l'article hygiénique est prévu pour l'utilisation dans le cas d'une incontinence grave et/ou légère.

8. Procédé pour la préparation d'articles hygiéniques, **caractérisé en ce qu'**on utilise lors de la préparation au moins une composition définie dans les revendications 1 à 4.
